# EUROPEAN PATENT SPECIFICATION

(11) **EP 4 294 781 B1**
(45) Date of publication and mention of the grant of the patent: **20.05.2026**
(21) Application number: 22708100.7
(22) Date of filing: 21.02.2022
(51) Int. Cl.: C07B 39/00, C07B 47/00, C07C 41/01, C07C 43/164, C07C 67/00, C07D 213/73, C07C 51/367, C07C 67/31, C07C 45/64, C07C 41/05, C07B 41/04

(54) **CATALYTIC METHOD FOR THE PREPARATION OF PERFLUOROALKOXY-SUBSTITUTED ARENES AND HETEROARENES**
KATALYTISCHES VERFAHREN ZUR HERSTELLUNG VON PERFLUORALKOXYSUBSTITUIERTEN ARENEN UND HETEROARENEN
PROCÉDÉ CATALYTIQUE DE PRÉPARATION D'ARÈNES ET D'HÉTÉROARÈNES SUBSTITUÉS DE PERFLUOROALCOXY

(30) Priority: 22.02.2021 EP 21158495
(43) Date of publication of application: 27.12.2023
(73) Proprietor: Freie Universität Berlin, 14195 Berlin (DE)
(72) Inventor: HASENSTAB-RIEDEL, Sebastian, 14532 Kleinmacknow (DE); HOPKINSON, Matthew, 14167 Berlin (DE); DIX, Stefan, 10319 Berlin (DE); GOLZ, Paul, 15234 Frankfurt (DE); SCHMID, Jonas Rachid, 10781 Berlin (DE); PERNICE, Holger, 28790 Schwanewede (DE); GUTMANN, Sebastian, 30173 Hannover (DE)
(74) Representative: Maikowski & Ninnemann Patentanwälte Partnerschaft mbB
(86) International application number: PCT/EP2022/054181
(87) International publication number: WO 2022/175508

(56) References cited:
- US-A1- 2021 032 181
- ZHENG WEIJIA ET AL: "Redox-Active Reagents for Photocatalytic Generation of the OCF 3 Radical and (Hetero)Aryl C-H Trifluoromethoxylation", vol. 57, no. 42, 20 September 2018 (2018-09-20), DE, pages 13795 - 13799, XP055832592, ISSN: 1433-7851, Retrieved from the Internet <URL:https://onlinelibrary.wiley.com/doi/full-xml/10.1002/anie.201808495> DOI: 10.1002/anie.201808495
- ZHENG WEIJIA ET AL: "Catalytic C-H Trifluoromethoxylation of Arenes and Heteroarenes", vol. 57, no. 31, 13 March 2018 (2018-03-13), DE, pages 9645 - 9649, XP055832594, ISSN: 1433-7851, Retrieved from the Internet <URL:https://onlinelibrary.wiley.com/doi/full-xml/10.1002/anie.201800598> DOI: 10.1002/anie.201800598
- TOY M S ET AL: "PHOTOCHEMICAL REACTIONS OF PERFLUOROOIALKYL PEROXIDES WITH PERFLUORO- CYCLOOLEFINS", J. FLUOR. CHEM., vol. 7, 1 January 1976 (1976-01-01), pages 375 - 383, XP055832597
- DIX STEFAN ET AL: "Radical C-H Trifluoromethoxylation of (Hetero)arenes with Bis(trifluoromethyl)peroxide", CHEMISTRY - A EUROPEAN JOURNAL, vol. 27, no. 45, 7 June 2021 (2021-06-07), DE, pages 11554 - 11558, XP055923130, ISSN: 0947-6539, Retrieved from the Internet <URL:https://onlinelibrary.wiley.com/doi/full-xml/10.1002/chem.202101621> DOI: 10.1002/chem.202101621
- PELAEZ W J ET AL: "Co-thermolysis: a one-pot synthetic method for novel 2-substituted-5-(trifluoromethoxy)thiophenes", TETRAHEDRON LETTERS, ELSEVIER, AMSTERDAM , NL, vol. 51, no. 40, 6 October 2010 (2010-10-06), pages 5242 - 5245, XP027252364, ISSN: 0040-4039, [retrieved on 20100725]

## Description

This application claims priority filed on 22 February 2021 in EUROPE with Nr 21158495.8.

### Technical field

The present invention relates to methods for the direct C-H perfluoroalkoxylation of arenes and heteroarenes.

### Background

Fluorinated organic compounds often have interesting pharmacological properties. The introduction of fluorine atoms and fluorinated organic groups therefore plays an important role in the development of pharmaceutical and agrochemical targets as well as for the provision of building blocks useful in the synthesis of such compounds.

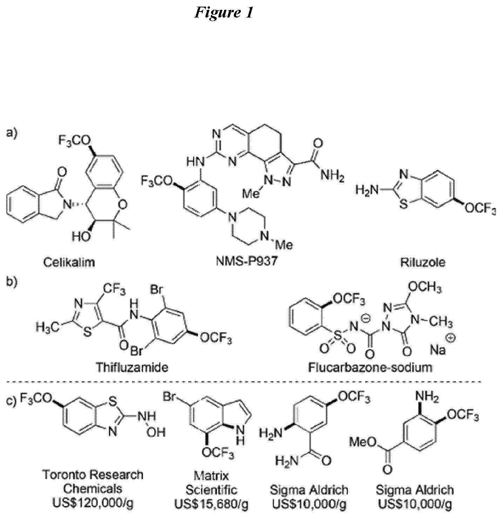

Among the fluorine containing functional groups, perfluoroalkoxy groups, in particular the trifluoromethoxy (OCF₃) group, is of great interest because of unique structural and electronic properties which can be useful not only in pharmaceutical, but also in materials and agricultural sciences. Examples of important OCF₃-substituted a) pharmaceuticals, b) agrochemicals and c) building blocks known in the art are shown in **Figure 1****.**

Yet, the introduction of perfluoroalkoxy groups into organic molecules is challenging. There are only very limited possibilities for the synthesis of perfluoroalkoxy-substituted molecules. Methods often suffer from poor substrate scope and/or require a multi-step sequence in order to finally arrive at the perfluoroalkoxy-substituted target compound.

Known approaches for the introduction of perfluoroalkoxy groups into organic molecules include the *de novo* construction of the perfluoroalkoxy group in the molecule; the indirect perfluoroalkylation of aliphatic and (hetero)aromatic alcohols by means of reaction with an electrophilic perfluoroalkyl donor; and the direct perfluoroalkoxylation of organic molecules using a perfluoroalkoxy donor reagent (for a minireview on "Synthetic approaches to Trifluoromethoxy-Substituted Compounds", see: A. Tlili et al., Angew. Chem. Int. Ed. 2016, 55, 11726-11735). Limitations for the *de novo* construction of trifluoromethoxylated pyridines are that hydroxypyridines are used as substrates and that the final halogen exchange step is performed with stoichiometric SbF₃. Most importantly, this method can only be applied on *ortho*-chloro pyridines (B. Manteau et al. "A General Approach to (Trifluoromethoxy)pyridines: First X-ray Structure Determinations and Quantum Chemistry Studies", Eur. J. Org. Chem. 2010, 6043-6066). Any other functionality, such as nitrile or amine functionalities, therefore has to be introduced in a multi-step procedure that often includes expensive palladium catalyzed reactions.

*Bis*-perfluoroalkylperoxides, such as e.g. *bis*-trifluoromethylperoxide (CF₃OOCF₃ or (OCF₃)₂) and *bis*-perfluoro-*tert*-butylperoxide ((CF₃)₃COOC(CF₃)₃ or (OC(CF₃)₃)₂), have been used as perfluoroalkoxy donor reagents in a photochemical reaction with perfluorocycloolefins (M. S. Toy et al, Journal of Fluorine Chemistry, 7 (1976) 375-383). In that case, a strong ultraviolet source was required for the homolytic fragmentation of the peroxide in order to form the perfluoroalkoxy radical which then added to the olefin bond of the perfluorocycloolefin substrate.

*Bis*-trifluoromethylperoxide has also been used as a donor reagent for the addition of OCF₃ to the substrate CF=CFSF₅, where the CF₃O radical was generated either thermally by heating the reactants at 185°C for 15 hrs, or by UV-photolysis by irradiating the reactants for one day at ambient temperature (E. F. Witucki, "Addition of Fluoroperoxides to Perfluorovinylsulfur Pentafluoride", Journal Fluorine Chemistry 20 (1982) 807-811).

Bis-trifluoromethylperoxide has furthermore been used for the trifluoromethoxylation of 2-substituted thiophenes (W.J. Piláes, "Co-thermolysis: a one-pot synthetic method for novel 2-substituted 5-(trifluoromethoxy)thiophenes", Tetrahedron Letters 51 (2010) 5242-5245). However, this reaction also requires relatively harsh conditions as it is carried out at 200 °C in the gas phase.

Due to the harsh conditions required and the poor substrate scope in the direct perfluoroalkoxylation of organic molecules with *bis-*perfluoroalkylperoxides, such as *bis*-trifluoromethylperoxide (OCF₃)₂, these reagents are commonly considered as having limited utility in the perfluoroalkoxylation of organic molecules including arenes and heteroarenes. Probably in view of this situation, the company Air Products and Chemicals, Inc., which has a patent on the preparation of high purity fluorinated peroxides including *bis*-trifluoromethylperoxide (EP 1 757 581), has discontinued the commercial distribution of *bis*-trifluoromethylperoxide (J. Jelier et al., "Radical Trifluoromethoxylation of Arenes Triggered by a Visible-Light-Mediated N-O Bond Redox Fragmentation", Angew. Chem. Int. Ed. 2018, 57, 13784-13789, reference [13]).

Alternative approaches for the direct introduction of the OCF₃ group have been developed. For example, in the above-mentioned publication of J. Jelier et al., a trifluoromethoxy transfer reagent derived from the trifluoromethylation of pyridine N-oxides is used for the catalytic trifluoromethylation of arenes triggered by light-mediated N-O bond redox fragmentation. However, the preparation of the trifluoromethoxy transfer reagent used in J. Jelier et al. involves the "Togni Reagent I" (3,3-Dimethyl-1-(trifluoromethyl)-1,2-benziodoxol), which is relatively expensive. Accordingly, trifluoromethoxy transfer reagents that need to be produced with "Togni Reagent I" are less attractive for industrial applications. Similar considerations apply to trifluoromethoxy transfer reagents prepared from Togni Reagent II (1-(Trifluoromethyl)-1,2-benzoiodoxol-1(1*H*)-on.

Hence, if methods benefiting from an enhanced practicability of *bis-*perfluoroalkylperoxides as perfluoroalkoxy donors were available, these would serve as valuable tools for the direct perfluoroalkoxylation of organic molecules. It would also be desirable if such methods allowed access to a variety of perfluoroalkoxylated substrates, including perfluoroalkoxylated arenes and heteroarenes.

### Summary of the present invention

The present invention addresses these needs by the provision of methods according to the following items:
[1] Method for the preparation of (CₙF₂ₙ₊₁)O-substituted arenes and heteroarenes **characterized in that**
   a peroxide reagent according to the following general formula (I)

      (OCₙF₂ₙ₊₁)₂ (I),
   is fragmented in the presence of an electron transferring catalyst under (CₙF₂ₙ₊₁)O-radical formation, and said (CₙF₂ₙ₊₁)O-radical then substitutes a C-H bond of an arene or heteroarene with a (CₙF₂ₙ₊₁)O-group,
   wherein in the above formulae n is an integer in the range from 1 to 4.
[2] Method for the direct perfluoroalkoxylation of arenes and heteroarenes with a (CₙF₂ₙ₊₁)O-group, **characterized in that**
   a peroxide reagent according to the following general formula (I)

      (OCₙF₂ₙ₊₁)₂ (I),
   is fragmented in the presence of an electron transferring catalyst under (CₙF₂ₙ₊₁)O-radical formation, and said (CₙF₂ₙ₊₁)O-radical then substitutes a C-H bond of an arene or heteroarene with a (CₙF₂ₙ₊₁)O-group,
   wherein in the above formulae n is an integer in the range from 1 to 4.

The invention further concerns methods for the preparation of pharmaceutical or agrochemical compounds or building blocks for the synthesis of pharmaceutical and agrochemical compounds, comprising the methods described above.

### Detailed description of the present invention

In the context of the present invention, the singular form is intended also to include the plural, for example, the term "catalyst" includes mixtures of two or more catalysts. All aspects and embodiments of the present invention are combinable. In the context of the present invention, the term "comprising" is intended to include the meaning of "consisting of" and "consisting essentially of". Endpoints of ranges are also included in the disclosure, e.g. a temperature range of 20°C to 30°C includes the values 20°C and 30°C.

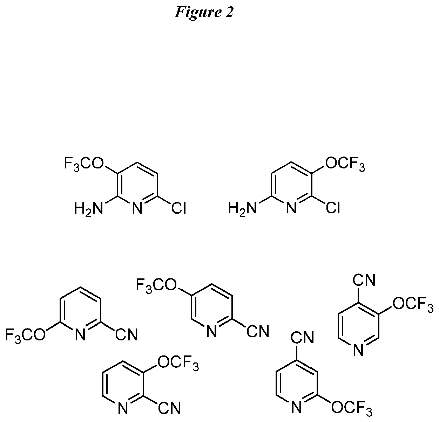

The present invention uses a catalytic approach for the direct perfluoroalkoxylation of arenes and heteroarenes, and the preparation of (CₙF₂ₙ₊₁)O-substituted arenes and heteroarenes, respectively (wherein in this formula, n is an integer in the range from 1 to 4). The methods of the present invention allow access to versatile perfluoroalkoxylated arenes and heteroarenes, which may be useful as building blocks in the synthesis of pharmaceutical and agrochemical target compounds, in one step. Such building blocks of commercial interest e.g. include trifluoromethoxy-substituted pyridines as shown in **Figure 2****,** which are amino-, halo- and/or cyano-substituted pyridines. The building blocks shown in **Figure 2** are directly accessible in one step from the corresponding non-trifluoromethoxylated pyridines following the methods of the present invention, and can be used for further functionalization, e.g. for amide formation (from the amino group), for ipso-substitution of the chloro group, for cyclization of the nitrile group, and for hydrolyzation of the nitrile group into a carboxyl-group (which, again, can be used for further functionalization, e.g. amide formation).

WO 2011/044181 A1, for example, discloses in Scheme 25 (step 1) and 26 (step 1 - 4) the preparation of 5-trifluoromethoxy picolinic acid, which is further transferred into a pharmaceutically active compound (designated as Example 123 in WO 2011/044181 A1). 5-trifluoromethoxy picolinic acid is prepared from 2-cyano-5-trifluoromethoxy pyridine, which can be obtained in one step from 2-cyanopyridine following the methods of the present invention. In WO 2011/044181 A1, a four step sequence starting from 2-chloro-5-hydroxy pyridine is required for the preparation of 2-cyano-5-trifluoromethoxy pyridine. Following the methods of the present invention, only one step is required, and hence less reactants, solvents etc. for the preparation of the same compound. Therefore, the methods of the present invention provide economic and ecological advantages over the reaction sequence applied in WO 2011/044181 A1 for the preparation of 2-cyano-5-trifluoromethoxy pyridine.

For carrying out the methods of the present invention, typically a mixture of (hetero)arene, catalyst and, optionally, an additive is provided, optionally in a solvent. Generally, this mixture is cooled (typically using liquid nitrogen) and the peroxide reagent according to the general formula (I) is added to the reaction mixture. The mixing of the reagents at this low temperature can be advantageous for practical reasons, as some of the reagents, such as (OCF₃)₂, are gaseous under ambient condition. Often, after the addition of the peroxide reagent, the reaction mixture is allowed to warm up to room temperature (which for the purpose of the present invention is defined as a temperature between 20 and 30°C) and allowed to stir until the conversion is complete. Advantageously,the reaction is carried out under protective gas, such as e.g. nitrogen or argon. The foregoing is not intended to limit the methods; other sequences of addition of reactants, solvents, catalyst and additives as well as temperatures are also feasible. The methods can be carried out batch-wise or in a continuous procedure.

The perfluoroalkoxylated arenes and heteroarenes resulting from the methods of the present invention in some instances are obtained as a mixture of regioisomers (of course only in those cases where the formation of regioisomers is possible). For the purification of the reaction mixture and the isolation of the perfluoroalkoxylated products, purification techniques known to the skilled person, such as chromatographic methods (e.g. column chromatography on a laboratory scale or HPLC) and distillation methods (including distillations on an industrial scale), can be feasibly used.

"Direct perfluoroalkoxylation" in the context of the present invention is defined in accordance with the common understanding of the skilled person, namely that the (CₙF₂ₙ₊₁)O-group is introduced by means of a (CₙF₂ₙ₊₁)O-donor reagent (wherein in these formulae, n is an integer in the range from 1 to 4). In the present case, the donor reagent is the peroxide reagent according to the general formula (I): (OCₙF₂ₙ₊₁)₂ (I), wherein n is an integer in the range from 1 to 4. The methods of the present invention improve the usefulness of *bis-*perfluoroalkylperoxides according to the general formula (I) as a reagent for the direct perfluoroalkoxylation of arenes and heteroarenes, and allow the preparation of (CₙF₂ₙ₊₁)O-substituted arenes and heteroarenes in one step (wherein in this formula, n is an integer in the range from 1 to 4).

The peroxide reagents suitable for use in the present invention include (OCF₃)₂, (OC₂F₅)₂, (O*-n*-C₃F₇)₂, (O-*iso*-C₃F₇)₂, (O-*n*-C₄F₉)₂, (O-*iso*-C₄F₉)₂ and (O-*tert-*C₄F₉)₂*,* thus reflecting all integers 1, 2, 3 and 4 = n, wherein (OCF₃)₂ and (O*-tert*-C₄F₉)₂ are preferred peroxide reagents for use in the present invention. The skilled person is familiar with methods for the preparation of these peroxide reagents. A method for the preparation of (OCF₃)₂ is e.g. disclosed in EP 1 757 581.

The methods of the present invention hence allow access to CF₃O-, C₂F₅O-, *n*-C₃F₇O-, *iso*-C₃F₇O, *n*-C₄F₉O-, *iso*-C₄F₉O, *tert*-C₄F₉O-substituted arenes and heteroarenes, with CF₃O- and *tert*-C₄F₉O-substituted arenes and heteroarenes being preferred target compounds.

The methods according to the present invention are catalytic methods which use an "electron transferring catalyst" for the C-H substitution of arenes and heteroarenes with a perfluoroalkoxy-group derived from the peroxide reagent according to the general formula (I).

"Electron transfer" in accordance with the common understanding of the skilled person means the relocation of an electron from a chemical entity being an atom, ion or molecule to another such chemical entity, which results in a change of oxidation state of the chemical entities involved. Electron transfer can also occur between an electrode and a chemical entity.

A "catalyst" for the purpose of the present invention is defined in accordance with the common understanding of the skilled person in the art, i.e. it is a substance, which increases the rate of a chemical reaction by lowering the activation barrier. The catalyst is not consumed by means of the catalyzed reaction but can act repeatedly in said reaction. In the present invention, this means that the method is carried out preferably with substoichiometric amounts of the catalyst, however, it is also possible to carry out the methods of the present invention using stoichiometric amounts of the catalyst.

Without being bound to the theory, it is assumed that the electron transferring catalyst used in the present invention transfers an electron to the peroxide reagent according to the general formula (I), which results in the fragmentation of the peroxide reagent and the formation of a (CₙF₂ₙ₊₁)O-radical and an (CₙF₂ₙ₊₁)O-anion (wherein in both the radical species and the anion species, n is an integer in the range from 1 to 4). The (CₙF₂ₙ₊₁)O-radical presumably then reacts with the arene or heteroarene substrate by means of formal C-H substitution, which means that a C-H bond of the arene or heteroarene is formally substituted with a (CₙF₂ₙ₊₁)O-group, thereby providing perfluoroalkoxy-substituted arenes or heteroarenes in a (formal) radical C-H substitution reaction (wherein in these formulae n is an integer in the range from 1 to 4). The reaction is overall redox-neutral as an electron is transferred back to the catalyst in the course of the catalytic cycle.

Suitable electron transferring catalysts for use in the methods of the present invention generally include metal compounds, i.e. (transition) metal salts and transition metal coordination complexes; stable aminoxyl radical compounds, and organic dyes.

The definition of a "(transition) metal salt" for the purpose of the present invention is in accordance with the common understanding of the skilled person, i.e. a (transition) metal salt is an ionic assembly of (transition) metal cations and organic or inorganic anions, such as e.g. halides (F⁻, Cl⁻, Br⁻, I⁻), sulfate (SO₄²⁻), phosphate (PO₄³⁻), pentafluorosulfate (SF₅⁻), hexafluorophosphate (PF₆⁻), cyanide (CN⁻), Thiocyanate (SCN). "(transition) metal salts" intends to denote the group of metal salts, comprising the more specific group of transition metal salts. The term "transition metal" intends to denote a metal selected from groups 3 to 12 of the periodic system.

While metal salts suitable for use as the electron transferring catalyst in the methods of the present invention include main group III metal salts (preferably aluminum salts, more preferably aluminum halides including, but not limited to AlCl₃), transition metal salts are preferred as electron transferring catalyst over main group metal salts. In this regard, it is noted that salts derived from alkali metal and earth alkali metals (such as e.g. Na₂SO₄, KCl, MgCO₃ etc.) are not suitable for use as electron transferring catalyst in the methods of the present invention.

Transitions metal salts preferably used as electron transferring catalyst in the methods of the present invention include salts from e.g. Cu, Fe, Ti, which also includes salts of the these metals at different oxidation states.

Examples for transition metal salts for use as the electron transferring catalyst in the methods of the present invention include, but are not limited to Cu(I) salts such as Cu₂O, CuCl, CuCl₂, CuBr, CuI, CuSCN; Cu(II) salts such as CuSO₄, CuCO₃; Fe(II) salts such as FeSO₄; Ti(III) salts such as TiCl₃.

The definition of a "transition metal coordination complex" for the purpose of the present invention is also in accordance with the common understanding of the skilled person, i.e. it consists of a central atom or ion (coordination center) bound by an array of molecules or ions (ligands). The transition metal coordination complex may be neutral or it may form the anionic or cationic part of a salt. Some transition metal coordination complex compounds have the ability to absorb visible light, thereby getting into an activated form suitable for electron transfer.

Transitions metal coordination complexes compounds preferably used as electron transferring catalyst in the methods of the present invention include coordination complex compounds from Cu, Ru and Ir, which also includes coordination complexes of these metals at different oxidation states.

Examples for transition metal coordination complex compounds for use as the electron transferring catalyst in the methods of the present invention include, but are not limited to Cu(I) compounds such as Cu(MeCN)₄PF₆; Ru(II) compounds such as Ru(bpy)₃(PF₆)₂ (wherein bpy = 2,2'-bipyridine); Ir(III) compounds such as [Ir(dF(CF₃)ppy)₂(dtbbpy)]PF₆ (wherein dF(CF₃)ppy = 2-(2,4-difluorophenyl)-5-(trifluoromethyl)pyridine; dtbbpy = 4,4'-di-*tert*-butyl-2,2'-bipyridine) and *fac*-Ir(ppy)₃ (wherein ppy = 2-phenylpyridine). Ru(bpy)₃(PF₆)₂ is particularly preferred as the electron transferring catalyst in the methods of the present invention.

Ru(bpy)₃(PF₆)₂, Ir(ppy)₃, [Ir(dF(CF₃)ppy)₂(dtbbpy)]PF₆ can be prepared according to literature procedures (D. Hanss, J. C. Freys, G. Bernandinelli, O. S. Wenger, *Eur. J. Inorg. Chem.* **2009,** 4850).

Stable aminoxyl radical compounds act as redox-active agents and are therefore suitable for use as electron transferring catalyst in the methods of the present invention. Examples for such compounds that have been found to be active as an electron transferring catalyst in the methods of the present invention are the stable aminoxyl radical compound 2,2,6,6-tetramethylpiperidinyloxyl ("TEMPO") and derivatives thereof, including, but not limited to 4-hydroxy-TEMPO, i.e. 4-hydroxy-2,2,6,6-tetramethylpiperidinyloxyl ("TEMPOL").

An "organic dye" for the purpose of the present invention is an organic compound, which has the ability to absorb visible light, thereby getting into an activated form suitable for electron transfer. Examples of organic dyes useful as electron transferring catalyst in the methods of the present invention include e.g. Fluorescein, Eosin (such as e.g. Eosin Y) and 9-mesityl-10-methylacridinium perchlorate.

Depending on the electron transferring catalyst used, the methods of the present invention are carried out in the absence of light irradiation, preferably induced thermally, or photocatalytically (i.e. under light irradiation).

In the photocatalytic embodiment, an electron transferring catalyst is used, which has the capability to absorb light, thereby getting into an activated state suitable for electron transfer In that case, the electron transferring catalyst represents a "photocatalyst".

In the other embodiment, the catalytic activity of the electron transferring catalyst does not depend on light irradiation. In this embodiment, the catalytic activity preferably is induced thermally. In these cases, the methods of the present invention are catalytic, but not photocatalytic methods. "Induced thermally" intends to denote that the reaction is performed at a temperature which is at least sufficient to induce the catalytic activity. This means that the reaction can be performed by heating the mixture to the reaction temperature, but, depending on the selected reagents and catalyst, the reaction can also proceed at room temperature or below room temperature. Although this embodiment does not depend on light irradiation, this does not intend to mean that any light source must be excluded during the methods according to this embodiment.

Due to their ability to absorb visible light, transition metal coordination complexes and organic dyes as described above are preferably used as photocatalysts in the present invention, i.e. the methods are then carried out under light irradiation in order to bring the catalyst in an activated state suitable for electron transfer. Transition metal coordination complexes are more preferred than organic dyes for use as photocatalysts in the present invention.

Transition metal coordination complexes suitable as photocatalysts in the methods of the present invention include Ru(II) compounds such as Ru(bpy)₃(PF₆)₂; Ir(III) compounds such as [Ir(dF(CF₃)ppy)₂(dtbbpy)]PF₆ and *fac*-Ir(ppy)₃; wherein Ru(bpy)₃(PF₆)₂ is a particularly preferred photocatalyst for use in the methods of the present invention.

The light used for activation of the photocatalyst in the photocatalytic embodiment of present invention is preferably visible light. For the purpose of the present invention, "visible light" is defined as having a wavelength λ ranging from 380 nm to 700 nm, including violet light (with λ typically being in the range from 380 nm to <435 nm), blue light (with λ typically being in the range from 435 nm to <500 nm), cyan light (with λ typically being in the range from 500 nm to <520 nm), green light (with λ typically being in the range from 520 nm to <565 nm), yellow light (with λ typically being in the range from 565 nm to <590 nm), orange light (with λ typically being in the range from 590 nm to <625 nm) and red light (with λ typically being in the range from 625 nm to 700 nm). In a more preferred embodiment, the visible light is selected from the group consisting of violet light, blue light, cyan light and green light for the irradiation.

Practically, the irradiation in the methods of the present invention can be carried out using light emitting diodes covering different wavelength ranges and typically indicating a wavelength maximum (λₘₐₓ). For blue light irradiation, an LED with a λₘₐₓ of 440 nm can for example be used.

In general, the methods of the present invention are preferably carried out photocatalytically using a transition metal coordination complex as described above, particularly Ru(bpy)₃(PF₆)₂, as the electron transferring catalyst (photocatalyst), or non photocatalytically, preferably thermally, using a stable aminoxyl radical compound as described above, particularly TEMPO, as the electron transferring catalyst.

Substrates suitable for use in the present invention include arene and heteroarene compounds, which are transferred into perfluoroalkoxy-substituted arenes and heteroarenes following the methods of the present invention, wherein CF₃O- and *tert*-C₄F₉O-substituted arenes and heteroarenes are preferred, with CF₃O-substituted arenes and heteroarenes being particularly preferred.

In accordance with the common understanding of the skilled person, "arenes" for the purpose of the present invention are defined as aromatic hydrocarbons, such as benzenes and naphthalenes.

In accordance with the common understanding of the skilled person, "heteroarenes" for the purpose of the present invention are aromatic hydrocarbons, wherein at least one carbon atom is replaced by a heteroatom such as nitrogen, sulfur or oxygen. Heteroarenes suitable for use in the present invention include, but are not limited to pyridines, furans, pyrroles, thiophenes, pyrazoles, imidazoles, benzimidazoles, indoles, quinolines, isoquinolines, purines, pyrimidines, thiazoles, pyrazines, oxazoles and triazoles. Pyridines are preferred heteroarenes for use in the present invention, particularly aminopyridines, halopyridines and cyanopyridines.

The arenes and heteroarenes for use in the present invention can be substituted with one or more functional groups, including, but not limited to:
lower alkyl (i.e. C1-C6 alkyl), including linear, branched and cyclic isomers, such as e.g. methyl, ethyl, *n*-propyl, *iso*-propyl, *n*-butyl, *iso*-butyl, *tert-*butyl, *n*-pentyl, *iso-*pentyl, *cyclo*-pentyl, *n*-hexyl, *cyclo*-hexyl;
higher alkyl (i.e. C7-C12 alkyl), including linear, branched and cyclic isomers, such as e.g. *n*-heptyl, *cyclo*-heptyl, *n*-octyl, *cyclo*-octyl, *n*-nonyl, *n-*decyl, *n*-dodecyl;
halides, including F, Cl, Br, I;
oxygen containing functional groups, including, but not limited to hydroxyl, carbonyl, aldehyde, carboxyl, carboalkoxy (e.g. carbomethoxy, carboethoxy, carbo-*iso*-propoxy, carbo-*tert*-butoxy), alkoxy (e.g. methoxy, ethoxy, *iso*-propoxy, *tert*-butoxy), silyloxy;
nitrogen containing functional groups, including but not limited to amide, amine, nitrile, nitro;
sulfur containing functional groups, including but not limited to: thiol, thioether, sulfoxide, sulfone, thiocyanate;
boron containing functional groups, including but not limited to boronic acids, boronic acid esters.

The arenes and heteroarenes for use in the present invention as well as the functional groups described above can in principle also be substituted with one or more aromatic and heteroaromatic functional groups including, but not limited to phenyl, benzyl, pyridinyl, furanyl, pyrrolyl, thiophenyl, pyrazolyl, imidazolyl, benzimidazolyl, indolyl, quinolinyl, isoquinolinyl, purinyl, pyrimidinyl, thiazolyl, pyrazinyl, oxazolyl and triazolyl. However, in that case, it needs to be kept in mind that such groups may also react with the peroxide reagent in the methods of the present invention. Therefore, it is preferred in the present invention that the arene and heteroarenes used as substrates in the methods of the present invention are not substituted with aromatic and heteroaromatic functional groups, or with functional groups that contain aromatic or heteroaromatic moieties. Similar considerations apply to functional groups that contain olefin or acetylene moieties.

Preferred arenes for use in the methods of the present invention include, but are not limited to benzene and derivatives thereof, fluorobenzene and derivatives thereof; chlorobenzene and derivatives thereof; bromobenzene and derivatives thereof; benzonitrile and derivatives thereof; benzaldehyde and derivatives thereof; benzoic acid and derivatives thereof; benzoic acid ester (such as e.g. benzoic acid methyl ester) and derivatives thereof; benzamide and derivatives thereof; phenyl boronic acid and derivatives thereof; wherein "derivatives thereof" means that one or more further functional groups as described above can be present in the arene compound.

(Hetero)arenes are preferred substrates in the photocatalytic embodiment of the present invention, especially if a transition metal coordination complex, such as Ru(bpy)₃(PF₆)₂, is used as the electron transferring catalyst (photocatalyst); and/or (OCF₃)₂ is used as the peroxide reagent.

Preferred heteroarenes for use in the methods of the present invention are pyridines, especially if a stable aminoxyl radical compound, preferably TEMPO, is used as the electron transferring catalyst; and/or (OCF₃)₂ is used as the peroxide reagent.

Trifluoromethoxylated pyridines are highly attractive building blocks in pharmaceutical and agricultural chemistry, but access to trifluoromethoxylated pyridine building blocks is limited due to synthetic challenges. It has been found herein that the methods of the present invention, in particular those wherein TEMPO is used as the electron transferring catalyst, are particularly suitable in order to provide perfluoroalkoxylated, in particular trifluoromethoxylated pyridines in one step.

One advantage of using the methods of the present invention in the preparation of perfluoroalkoxylated, in particular trifluoromethoxylated pyridines, is that the electron deficient 4-position of the pyridine substrate is typically not substituted due to the electrophilic nature of the (CₙF₂ₙ₊₁)O-radical (wherein in this formula, n is an integer in the range from 1 to 4) formed in the fragmentation of the peroxide reagent according to formula (I). This facilitates the separation of possible regioisomers.

Preferred pyridine substrates for use in the present invention, particularly if TEMPO is used as the electron transferring catalyst and/or (OCF₃)₂ is used as the peroxide reagent, include, but are not limited to halopyridines, in particular 2-halopyridins (wherein "halo" means fluoro, chloro and bromo, preferably chloro and bromo), aminopyridines and cyanopyridines. Examples include, but are not limited to 2-bromopyridine, 2-bromo-6-methylpyridine, 2-chloropyridine, 2-fluoro-6-aminopyridine, 2-bromo-4-fluoropyridine, 2-cyanopyridine, 3-cyanopyridine, 4-cyanopyridine, 2-fluoro-6-cyanopyridine, 2-fluoro-5-cyanopyridine, 2-amino-6-fluropyridine, 2-amino-6-chloropyridine with 2-amino-6-chloropyridine and 2-cyanopyridine being particularly preferred. The perfluoroalkoxy-substituted pyridines, in particular the trifluoromethoxy-substituted pyridines resulting from these substrates all represent excellent building blocks, which are accessible in only one single step by means of the methods of the present invention.

In relation to the peroxide reagent, the (hetero)arene substrate is typically used in excess molar amounts in the methods of the present invention. Typical molar ratios of peroxide reagent according to formula (I) and (hetero)arene substrate in the methods of the present invention include 1:2, 1:3, 1:4, 1:5, 1:6, 1:7, 1:8, 1:9, 1:10, 1:11, 1:12, 1:13, 1:14 and 1:15.

Using excess molar amounts of the arene or heteroarene substrates in relation to the peroxide has the advantage that the yield is improved and over-substitution is strongly diminished. The ratio of peroxide reagent according to formula (I) and the arene or heteroarene substrate is thus a compromise between efficacy of the reaction and economical/ecological aspects. Taking this into consideration, it is preferred that the peroxide reagent and the arene or heteroarene substrate are used in molar ratios of 1:5, 1:6, 1:7, 1:8, 1:9, 1:10, 1:11 or 1:12, 1:13, 1:14, 1:15 with molar ratios of 1:5, 1:6, 1:7, 1:8 and 1:10 being more preferred. Multiple substitution products are typically only observed in trace amounts if at least 5 equivalents of (hetero)arene substrate is used in relation to the peroxide reagent. In any event, multiple substitution products can be separated from the mono-substituted product(s) by means of purification techniques known to the skilled person, such as chromatographic methods (e.g. column chromatography or HPLC) and distillation methods (including distillations on an industrial scale).

The methods of the present invention can be performed in the presence of an organic solvent. However, they can also be performed "neat", i.e. in the absence of solvent, in particular if the arene or heteroarene substrate is a liquid at the reaction temperature, for example room temperature.

In general, high concentrations of the arene or heteroarene substrate in the reaction mixture are favorable in terms of improving the yield of the substitution reaction according to the methods of the present invention. At some point, however, a very high substrate concentration may result in practical problems regarding the distribution of chemicals in the mixture, in particular if the substrate is a solid compound at the reaction temperature, for example room temperature. The reaction mixture may then become a very thick slurry, which leads to a non-ideal distribution of components, thereby reducing yield of the reaction and should therefore be avoided. If that is the case, use of an organic solvent is preferable.

An organic solvent in some cases may also be useful if the catalyst is not soluble in the (liquid) arene or heteroarene substrate.

If an organic solvent is used in the methods of the present invention, the concentration of the arene or heteroarene in the solvent is typically within the range of 0.1 mol/L to 10 mol/L, preferably 0.2 mol/L to 8 mol/L, more preferably 0.5 mol/L to 6 mol/L, even more preferably 1 mol/L to 5 mol/L, including concentrations of e.g. 1.5 mol/L, 2 mol/L, 2.5 mol/L, 3 mol/L, 3.5 mol/L, 4 and 4.5 mol/L.

Suitable organic solvents for use in the present invention are typically polar aprotic solvents including, but not limited to acetonitrile (MeCN), acetone, *N,N-*dimethylformamide (DMF), dimethylsulfoxide (DMSO), tetrahydrofuran (THF), diethylether, iso-propylether (IPE), methyl-tert-butylether (MTBE), 1,4-dioxane, ethyl acetate (EtOAc), dichloromethane (DCM), 1,2-dichloroethane (1,2-DCE), chloroform and mixtures thereof. Acetonitrile, acetone and dichloromethane are preferred solvents for use in the present invention, acetonitrile is particularly preferred. Where appropriate, mixtures of two or more solvents can be used for the methods according to the present invention.

The methods of the present invention can optionally be carried out in the presence of an additive, which is typically a salt additive, in particular an alkali or earth alkali metal salt additive. It is believed that the additive helps to regenerate TEMPO and enables a catalytic process. Where appropriate, mixtures of two or more additives can be used for the methods according to the present invention.

Suitable additives for use in the methods of the present invention include, but are not limited to alkali and earth alkali metal carbonates and hydrogen carbonates, such as e.g. Li₂CO₃, Na₂CO₃, K₂CO₃, MgCO₃, CaCO₃, LiHCO₃, NaHCO₃, KHCO₃; alkali and earth alkali metal sulfates, such as e.g. Li₂SO₄, Na₂SO₄, K₂SO₄, MgSO₄, CaSO₄; alkali and earth alkali metal phosphates, hydrogen phosphates and dihydrogen phosphates, such as e.g. Li₃PO₄, Li₂HPO₄, LiH₂PO4, Na₃PO₄, Na₂HPO₄, NaH₂PO₄, K₃PO₄, K₂HPO₄, KH₂PO₄, CaHPO₄; alkali and earth alkali metal halides such as e.g. LiF, NaF, KF, CsF, MgF₂, CaF₂, LiCl, NaCl, KCl, CsCl, MgCl₂, CaCl₂, LiBr, NaBr, KBr, Csbr, MgBr₂, CaBr₂, LiI, NaI, KI, CsI, MgI₂, and CaI₂; Alkali and earth alkali metal carbonates and fluorides are preferred additives for use in the methods of the present invention, in particular Na₂CO₃, K₂CO₃ and KF.

Typical amounts of catalysts used in the method of the present invention are within the range of 0.01 to 2 equivalents, preferably 0.05 to 1 equivalent, more preferably 0.1 to 0.5 equivalent, based on the molar amount of the peroxide reagent according to the general formula (I).

Catalyst loadings described herein generally refer to the molar amount of reactant that is present in lower molar amounts in the reaction mixture in relation to the other reactant. "Reactants" in the methods of the present invention mean the arene/heteroarene substrate on the one hand, and the peroxide reagent according to the general formula (I) on the other hand. Since the arene/heteroarene is typically used in molar excess amounts with respect to the peroxide reagent according to the general formula (I), catalyst loadings referred to herein are typically in relation to the molar amount of the peroxide reagent unless otherwise indicated. If for example 1 equivalent (or 100 mol-%) of the peroxide reagent is used, a catalyst loading of e.g. 10 mol-% is in relation to the molar amount of peroxide reagent.

The methods of the present invention can in principle be performed with high catalyst loadings of up to 1 equivalent (100 mol-%). In that case, the electron transferring catalyst may be considered as "mediating" the formal C-H substitution reaction of the present invention. For example, it has been found by the inventors that copper compounds, such as e.g. CuCl and Cu(MeCN)₄PF₆, are particularly useful as electron transferring catalyst at stoichiometric amounts in the methods of the present invention.

However, it is of course preferred that the electron transferring catalyst of the present invention is used as a "real catalyst" at substoichiometric amounts. Substoichiometric amounts of the electron transferring catalyst useful in the present invention include for example 50 mol-% and below, 40 mol-% and below, and 30 mol-% and below.

This includes amounts within the range of 0.01 mol-% to 30 mol-%, preferably within the range of 0.05 mol-% and 25 mol-%, more preferably within the range of 0.1 mol-% and 20 mol-%, even more preferably within the range of 0.5 mol-% and 18 mol-%, even more preferably within the range of 1 mol-% and 16 mol-%, even more preferably within the range of 1 mol-% and 14 mol-%, even more preferably within the range of 1 mol-% and 12 mol-%, even more preferably within the range of 1 mol-% and 10 mol-%, even more preferably within the range of 1 mol-% and 8 mol-%, including catalyst loadings of e.g. 1.5 mol-%, 2 mol-%, 2.5 mol-%, 3 mol-%, 3.5 mol-%, 4 mol-%, and 4.5 mol-%, 5.0 mol-%, 5.5 mol-%, 6.0 mol-%, 6.5 mol-%, and 7.5 mol-%.

Of course, the catalyst loading also depends on the particular catalyst used.

If for example a (transition) metal salt is used as the electron transferring catalyst in the methods of the present invention, catalyst loadings, as mentioned above, can be up to 1 eq. Preferably, however, catalyst loadings with transition metal salts are within the range of 5 mol-% to 50 mol%, more preferably within the range of 7.5 mol-% and 40 mol-%, even more preferably within the range of 10 mol-% and 30 mol-%.

Using a transition metal coordination complex as the electron transferring catalyst often allows lower catalyst loadings. Hence, with such compounds, the methods of the present invention are preferably carried out photocatalytically under visible light irradiation.

If a transition metal coordination complex, such as the particularly preferred Ru(bpy)₃(PF₆)₂, is used as the electron transferring catalyst in the methods of the present invention, catalyst loadings are preferably within range from 0.1 mol-% and 6 mol-%, including catalyst loadings of e.g. 0.25 mol-%, 0.5 mol-%, 0.75 mol-%, 1 mol-%, 1.25 mol-%, 1.5 mol-%, 1.75 mol-%, 2 mol-%, 2.25 mol-%, 2.5 mol-%, 2.75 mol-%, 3 mol-%, 3.25 mol-%, 3.5 mol-%, 3.75 mol-%, 4 mol-%, 4.25 mol-%, 4.5 mol-%, 4.75 mol-%, 5 mol-%, 5.25 mol-%, 5.5 mol-%, and 5.75 mol-%.

Catalyst loadings in the range of 1 mol-% and 4 mol-% are particularly preferred for Ru(bpy)₃(PF₆)₂ as the electron transferring catalyst. The methods of the present invention are then preferably carried out photocatalytically, i.e. under light irradiation, wherein the light is visible light, preferably selected from violet light, blue light, cyan light and green light; and/or with *bis-*trifluoromethylperoxide ((OCF₃)₂) as the peroxide reagent according to the general formulae (I); and/or with arene or pyridine substrates, optionally substituted with one or more functional groups as described above, wherein the pyridine substrates are preferably selected from the group consisting of aminopyridines, cyanopyridines and halopyridines.

If a stable aminoxyl radical compound, such as the particularly preferred TEMPO, is used as the electron transferring catalyst in the methods of the present invention, catalyst loadings are preferably within the range from 5 mol-% and 30 mol-%, including catalyst loadings of e.g. 7.5 mol-%, 10 mol-%, 12.5 mol-%, 15 mol-%, 17.5 mol-%, 20 mol-%, 22.5 mol-%, 25 mol-%, and 27.5 mol-%. In that case, the methods are preferably carried out with *bis-*trifluoromethylperoxide ((OCF₃)₂) as the peroxide reagent according to the general formulae (I); and/or with pyridine substrates, optionally substituted with one or more functional groups as described above, in particular with pyridine substrates selected from the group consisting of aminopyridines, cyanopyridines and halopyridines.

The methods according to the present invention can be carried out at a temperature which is suitably selected according to e.g. reactivity of the reagents, the catalysts, the solvents and/or the regioselectivity of the method. This includes temperatures within the range of 0 °C to 80 °C, preferably within the range of 10 °C and 50 °C, and more preferably within the range of 20 °C and 30 °C.

In an alternative embodiment of the present invention, the electron transferring catalyst can also be replaced by an electric current, for example, if the C-H substitution or direct perfluoroalkoxylation of an arene or heteroarene with a (CₙF₂ₙ₊₁)O-group (wherein in this formula, n is an integer in the range from 1 to 4) using a peroxide reagent according to the general formula (I) is carried out in an electrode-electrode interface. In that case, the peroxide reagent according to the general formula (I) is electrochemically fragmented under (CₙF₂ₙ₊₁)O-radical formation, e.g. by means of contacting the negatively charged electrode in an electrode-electrode interface, and said (CₙF₂ₙ₊₁)O-radical then substitutes a C-H bond of an arene or heteroarene with a (CₙF₂ₙ₊₁)O-group (wherein in the above formulae, n is an integer in the range from 1 to 4).

The advantage of this electrochemical alternative method of the present invention is that reagent waste is reduced, thereby providing a more economical and ecological version of the methods of the present invention.

The following examples are intended to illustrate the present invention without limiting its scope.

### Examples

### General

The reactions according to the following examples with a maximum scale of 1 mmol were conducted in pressure tubes "P26 0001", larger scales were conducted in pressure tubes "P26 0006" from FengTecEx GmbH. Dry Solvents were prepared by filtering purchased HPLC- or analytical grade solvents through Aluminium oxide and storing them over activated Molecular sieves (3 or 4 Å) for at least two days. The experiments were all conducted under argon, dry working techniques and/or degassed and/or dry solvents if stated. Blue light irradiation was provided by LED strips (λₘₐₓ = 440 nm, IP65) purchased from PB Versand GmbH (Type: 5630). A crystallization flask wrapped with the LED strips was used as a light reactor.

NMR spectra were acquired on a JEOL ECX 400 (400 MHz), JEOL ECP 500/ Bruker Avance 500 (500 MHz), Varian INOVA 600 (600 MHz) or a Bruker Avance 700 (700 MHZ) in CDCl₃, CD₃CN or ((CD₃)₂CO) as a solvent.

### Example 1: Photocatalytic trifluoromethoxylation of exemplary (hetero)arenes

A pressure tube was charged with a stir bar, Ru(bpy)₃(PF₆)₂ (1.5 mol%, 0.0075 mmol, 6.5 mg), MeCN (2.5 mL) and the (hetero)arene substrate (5 eq). The mixture was frozen with liquid N₂ and the tube was evacuated. Afterwards (CF₃O)₂ (1 eq, 0.5 mmol) was condensed into the tube and the tube was vented shortly with argon. The closed tube was gently shaken in water until the mixture melted and was stirred for 16 h at room temperature under irradiation from blue LEDs. Afterwards, the tube was carefully opened towards the rear side of the fume hood to release overpressure. The internal standard trifluorotoluene (1 eq, 61 µL) was added and the mixture was vigorously shaken. Up to 1 mL of the reaction mixture was filtered through diatomaceous earth and transferred into an NMR-tube for ¹⁹F-NMR-Analysis.

The product yields according to the following **Table 1** were calculated from the ¹⁹F-NMR Spectrum using trifluorotoluene as an internal standard.

**Table 1**

| **Entry** | **Arene 1a** | **Product** (¹⁹F NMR yield) | **Regioselectivity** (ortho-/meta-/para) |
|---|---|---|---|
| **1** | Benzene | 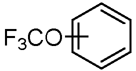 74%^{a} | - |
| | | (64%) | |
| **2** | Fluorobenzene | 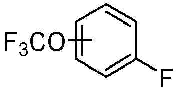 58%^{a} | 2.5/1/2.3^{a} |
| | | | 2.1/1/1.8 |
| | | 58% | |
| **3** | Chlorobenzene | 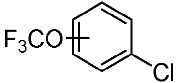 49% | 1.8/1/1.3 |
| **4** | Bromobenzene | 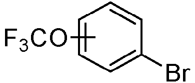 60%^{a} | 2.9/1/2.1^{a} |
| **5** | Benzonitrile | 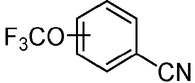 69%^{a} | 2.3/1.6/1^{a} |
| **6** | Benzaldehyde | 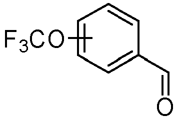 60%^{a} | 2.1/1.9/1^{a} |
| | | | 2.4/2.4/1 |
| | | 63% | |
| **7** | Benzoic acid | 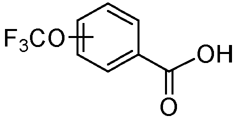 49%^{a} | 1.9/2/1^{a} |
| **8** | Benzoic acid methyl ester | 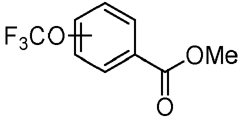 73%^{a} | 1.8/2.1/1^{a} |
| **9** | Acetophenone | 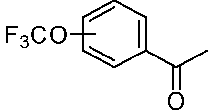 70%^{a} | 1.7/1.7/1^{a} |
| | | | 1.9/1.9/1 |
| | | 75% | |
| **10** | Phenylboronic acid pinacol ester | 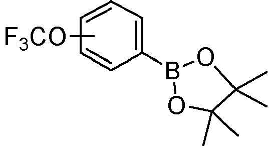 | 1.6/1.4/1 |
| **11** | Nitrobenzene | 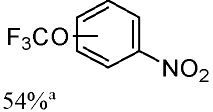 | 1/4.2/1.5^{a} |
| **12** | 2-amino-6-chloropyridine^{b} | 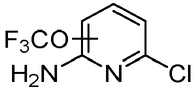 37% | n.d. |

| | | | |
|---|---|---|---|
| ^{a}*with 0.1 equiv KF* ^{b} *increased scale based on 3 mmol of (OCF₃)₂* | | | |

**Table 1** illustrates that trifluoromethoxy-substituted (hetero)arenes with a broad substrate scope can be obtained in moderate to good yields (37-75%) using the (photocatalytic) method of the present invention for their preparation.

### Example 2: TEMPO-catalyzed trifluoromethoxylation of exemplary arenes and pyridines

A pressure tube was charged with a stir bar, TEMPO (5 mol%, 0.025 mmol, 3.9 mg), K₂CO₃ (1 eq, 0.5 mmol, 69.1 mg) and the (hetero)arene substrate (5 eq). The mixture was frozen with liquid N₂ and the tube was evacuated. Afterwards, (CF₃O)₂ (1 eq, 0.5 mmol) was condensed into the tube and the tube was vented shortly with argon. The closed tube was gently shaken in water until the mixture melted and was stirred for 16 h at room temperature. Afterwards, the tube was carefully opened towards the rear side of the fume hood to release overpressure, the internal standard trifluorotoluene (1 eq, 61 µL) and MeCN (to achieve 0.2 M) was added and the mixture was vigorously shaken. Up to 1 mL of the reaction mixture was filtered through diatomaceous earth and transferred into an NMR-tube for ¹⁹F-NMR-Analysis.

The product yields according to the following **Table 2** were calculated from the ¹⁹F-NMR Spectrum using trifluorotoluene as an internal standard.

**Table 2**

| **Entry** | **Arene 2a** | **Product** | **Regioselectivity** |
|---|---|---|---|
| | | (¹⁹F NMR yield) | (ortho-/meta-/para) |
| **1** | Benzene | 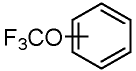 61% | - |
| **2** | Benzonitrile^{a} | 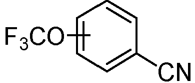 81% | 1.4/1.6/1 |
| **3** | Benzoic acid methyl ester | 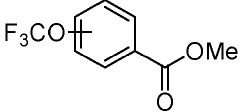 45% | 1/1.9/1.2 |
| **4** | 2-Chloropyridine | 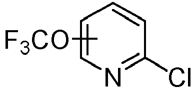 12% | n.d. |

| | | | |
|---|---|---|---|
| ^{a}*10 equivalents of arene were used* | | | |

**Table 2** illustrates that trifluoromethoxy-substituted arenes can be obtained in moderate to very good yields (Table 2, entries 1-3: 45-81%) using the (TEMPO-catalyzed) method of the present invention for their preparation.

**Table 2** also shows that under the chosen conditions, trifluoromethoxylation of 2-chloropyridine in principle occurs (Table 2, entry 4: 12%). This encouraging result led the inventors to the development of improved conditions for the trifluoromethylation of 2-halopyridines, which are important building blocks.

### Example 3: Improved conditions for the TEMPO-catalyzed trifluormethoxylation of exemplary pyridines

A pressure tube was charged with a stir bar, TEMPO (25 mol%, 0.125 mmol, 19.5 mg), Na₂CO₃ (1 eq, 0.5 mmol, 53 mg) and the pyridine substrate (5 eq). The mixture was frozen with liquid N₂ and the tube was evacuated. Afterwards (CF₃O)₂ (1 eq, 0.5 mmol) was condensed into the tube and the tube was vented shortly with argon. The closed tube was gently shaken in water until the mixture melted and was stirred 16 h at room temperature. Afterwards the tube was carefully opened towards the rear side of the fume hood to release overpressure. The internal standard trifluorotoluene (1 eq, 61 µL) and MeCN or Et₂O (to achieve 0.2 M) was added and the mixture was vigorously shaken. Up to 1 mL of the reaction mixture was filtered through diatomaceous earth and transferred into an NMR-tube for ¹⁹F-NMR-Analysis.

The product yields according to the following **Table 3** were calculated from the ¹⁹F-NMR Spectrum using trifluorotoluene as an internal standard.

**Table 3**

| **Entry** | **Pyridine 3a** | **Product** (¹⁹F NMR yield) | **Regioselectivity** |
|---|---|---|---|
| **1** | 2-Bromopyridine^{a} | 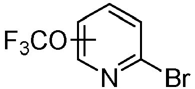 43% | n.d. |
| **2** | 2-Bromo-6-methylpyridin | 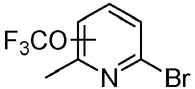 47% | n.d. |
| **3** | 2-chloropyridin^{b} | 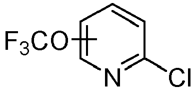 51% | n.d. |
| **4** | 2-Bromo-4-fluoropyridine | 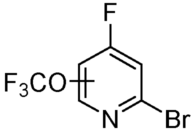 66% | n.d. |
| **5** | 2-Cyanopyridine^{b} | 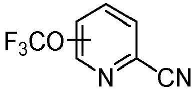 31% | n.d. |
| **6** | 3-Cyanopyridine^{c} | 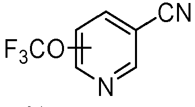 31% | n.d. |
| **7** | 4-Cyanopyridine^{d} | 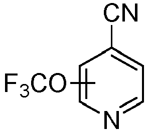 29% | n.d. |
| **8** | 2-amino-6-fluoropyridine^{e} | 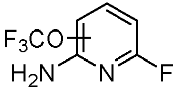 39% | n.d. |
| **9** | 2-amino-6-chloropyridine^{e} | 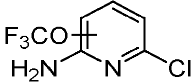 27 % | n.d. |

| | | | |
|---|---|---|---|
| ^{a} *10 mol% TEMPO, 65°C* ^{b} *increased scale based on 6 mmol of (OCF₃)₂* *^{c} increased scale based on 6 mmol of (OCF₃)₂ in 3 ml MeCN* *^{d} increased scale based on 6 mmol of (OCF₃)₂ in 30 ml MeCN* *^{e} 2.5 ml MeCN* | | | |

**Table 3** illustrates that trifluoromethoxy-substituted 2-halopyridines can be obtained in moderate to good yields (Table 3, entries 1-4: 43-66%) using the (TEMPO-catalyzed) method of the present invention for their preparation.

**Table 3** furthermore shows that trifluoromethoxy-substituted cyanopyridines (Table 3, entries 5-7) as well as the trifluoromethoxy-substituted 2-amino-6-fluoropyridine and 2-amino-6-chloropyridine (Table 3, entry 8-9) are also obtained following the (TEMPO-catalyzed) method of the present invention for their preparation, although with slightly lower yield (27-39%). Most importantly, only one reaction step is required in order to access those otherwise difficult to synthesize perfluoroalkoxylated pyridines that have the potential to serve as excellent building blocks.

### Example 4: CuCl-mediated trifluoromethoxylation of benzene

The pressure tube was charged with a stir bar, CuCl (1 eq, 0.5 mmol, 49.5 mg), MeCN (0.25 mL) and benzene (5 eq). The mixture was frozen with liquid N₂ and the tube was evacuated. Afterwards (CF₃O)₂ (1 eq, 0.5 mmol) was condensed into the tube and the tube was vented shortly with argon. The closed tube was gently shaken in water until the mixture melted and was stirred 16 h at room temperature. Afterwards the tube was carefully opened towards the rear side of the fume hood to release overpressure, because carbonyl fluoride is formed as a side product during the reaction. The internal standard trifluorotoluene (1 eq, 61 µL) and MeCN (to achieve 0.2 M) was added and the mixture was vigorously shaken. Up to 1 mL of the reaction mixture was filtered through diatomaceous earth and transferred into an NMR-tube for ¹⁹F-NMR-Analysis.

The product trifluoromethoxy benzene was obtained with 55% yield (calculated from the ¹⁹F-NMR Spectrum using trifluorotoluene as an internal standard).

Taking into consideration that perfluoroalkoxylated, in particular trifluoromethoxylated arenes and heteroarenes, including trifluoromethoxylated pyridines as a highly useful class of building blocks, are generally difficult to access, Examples 1-4 provided herein illustrate that the methods of the present invention serve as a valuable tool for the provision of such compounds with moderate to very good yields, thereby broadening the scope for the direct perfluoroalkoxylation of arene and heteroarene substrates.

## Claims

1. Method for the preparation of (CₙF₂ₙ₊₁)O-substituted arenes and heteroarenes **characterized in that**
a peroxide reagent according to the following general formula (I)
(OCₙF₂ₙ₊₁)₂ (I),
is fragmented in the presence of an electron transferring catalyst under (CₙF₂ₙ₊₁)O-radical formation, and said (CₙF₂ₙ₊₁)O-radical then substitutes a C-H bond of an arene or heteroarene with a (CₙF₂ₙ₊₁)O-group,
wherein in the above formulae n is an integer in the range from 1 to 4.

2. Method for the direct perfluoroalkoxylation of arenes and heteroarenes with a (CₙF₂ₙ₊₁)O-group, **characterized in that**
a peroxide reagent according to the following general formula (I)
(OCₙF₂ₙ₊₁)₂ (I),
is fragmented in the presence of an electron transferring catalyst under (CₙF₂ₙ₊₁)O-radical formation, and said (CₙF₂ₙ₊₁)O-radical then substitutes a C-H bond of an arene or heteroarene with a (CₙF₂ₙ₊₁)O-group,
wherein in the above formulae n is an integer in the range from 1 to 4.

3. Method according to any one of claims 1 and 2, wherein n is 1 or 4, preferably 1.

4. Method according to any one of claims 1 to 3, wherein the heteroarene is a pyridine, preferably a pyridine selected from the group of aminopyridines, halopyridines and cyanopyridines.

5. Method according to any one of claims 1 to 4, wherein the electron transferring catalyst is a (transition) metal salt, preferably a transition metal salt of Cu, Fe, Ti, more preferably a transition metal salt selected from the group consisting of Cu₂O, CuCl, CuCl₂, CuBr, CuI, CuSCN, CuSO₄, CuCO₃, FeSO₄, TiCl₃.

6. Method according to any one of claims 1 to 4, wherein the electron transferring catalyst is a stable aminoxyl radical compound, preferably a stable aminoxyl radical compound selected from the group consisting of 2,2,6,6-tetramethylpiperidinyloxyl ("TEMPO") and derivatives thereof.

7. Method according to any one of claims 1 to 6, wherein the method does not depend on light irradiation.

8. Method according to any one of claims 1 to 4, wherein the electron transferring catalyst is a transition metal coordination complex, preferably a transition metal coordination complex of Cu, Ru and Ir, more preferably a transition metal coordination complex selected from the group consisting of Cu(MeCN)₄PF₆, Ru(bpy)₃(PF₆)₂, [Ir(dF(CF₃)ppy)₂(dtbbpy)]PF₆ and Ir(ppy)₃.

9. Method according to claim 8, wherein the electron transferring catalyst is a photocatalyst and the method is carried out under light irradiation, wherein the light is visible light having a wavelength λ in the range from 380 nm to 700 nm.

10. Method according to any one of claims 8 and 9, wherein the electron transferring catalyst is selected from the group consisting of Ru(bpy)₃(PF₆)₂, [Ir(dF(CF₃)ppy)₂(dtbbpy)]PF₆ and Ir(ppy)₃, and is preferably Ru(bpy)₃(PF₆)₂.

11. Method according to any one of claims 1 to 10, wherein the electron transferring catalyst is used in substoichiometric amounts.

12. Method according to any one of claims 1 to 11, wherein the arene or heteroarene is used in excess molar amounts in relation to the peroxide reagent according to the general formula (I).

13. Method according to any one of claims 1 to 12, wherein the method is carried out in the presence of an organic solvent, wherein the solvent is a polar aprotic solvent selected from the group consisting of acetonitrile (MeCN), acetone, N,N-dimethylformamide (DMF), dimethylsulfoxide (DMSO), tetrahydrofuran (THF), diethylether, iso-propylether (IPE), methyl-tert-butylether (MTBE), 1,4-dioxane, ethylacetate (EtOAc), dichloromethane (DCM), 1,2-dichloroethane (1,2-DCE), chloroform and mixtures thereof; or in the absence of a solvent.

14. Method according to any one of claims 1 to 13, wherein the method is carried out in the presence of an additive, wherein the additive is a salt additive selected from the group consisting of alkali and earth alkali metal sulfates, alkali and earth alkali metal carbonates and hydrogen carbonates, alkali and earth alkali metal phosphates, hydrogen phosphates, and dihydrogen phosphates, and alkali and earth alkali metal halides.

15. Method for the preparation of pharmaceutical or agrochemical compounds or building blocks for the synthesis of pharmaceutical and agrochemical compounds, comprising the method according to anyone of claims 1 to 14.

## Patentansprüche

1. Verfahren zur Herstellung von (CₙF₂ₙ₊₁)O-substituierten Arenen und Heteroarenen, **dadurch gekennzeichnet, dass**
ein Peroxidreagens gemäß der folgenden allgemeinen Formel (I)
(OCₙF₂ₙ₊₁)₂ (I),
in Gegenwart eines Elektronen transferierenden Katalysators unter Bildung eines (CₙF₂ₙ₊₁)O-Radikals fragmentiert wird und das (CₙF₂ₙ₊₁)O-Radikal dann eine C-H-Bindung eines Arens oder Heteroarens mit einer (CₙF₂ₙ₊₁)O-Gruppe substituiert,
wobei in den obigen Formeln n eine ganze Zahl im Bereich von 1 bis 4 ist.

2. Verfahren zur direkten Perfluoralkoxylierung von Arenen und Heteroarenen mit einer (CₙF₂ₙ₊₁)O-Gruppe, **dadurch gekennzeichnet, dass**
ein Peroxidreagens gemäß der folgenden allgemeinen Formel (I)
(OCₙF₂ₙ₊₁)₂ (I),
in Gegenwart eines Elektronen transferierenden Katalysators unter Bildung eines (CₙF₂ₙ₊₁)O-Radikals fragmentiert wird und das (CₙF₂ₙ₊₁)O-Radikal dann eine C-H-Bindung eines Arens oder Heteroarens mit einer (CₙF₂ₙ₊₁)O-Gruppe substituiert,
wobei in den obigen Formeln n eine ganze Zahl im Bereich von 1 bis 4 ist.

3. Verfahren nach einem der Ansprüche 1 und 2, wobei n 1 oder 4 ist, vorzugsweise 1.

4. Verfahren nach einem der Ansprüche 1 bis 3, wobei das Heteroaren ein Pyridin ist, vorzugsweise ein Pyridin, das aus der Gruppe von Aminopyridinen, Halogenpyridinen und Cyanopyridinen ausgewählt ist.

5. Verfahren nach einem der Ansprüche 1 bis 4, wobei der Elektronen transferierende Katalysator ein (Übergangs-)Metallsalz ist, vorzugsweise ein Übergangsmetallsalz von Cu, Fe, Ti, mehr bevorzugt ein Übergangsmetallsalz, das aus der aus Cu₂O, CuCI, CuCl₂, CuBr, Cul, CuSCN, CuSO₄, CuCO₃, FeSO₄, TiCl₃ bestehenden Gruppe ausgewählt ist.

6. Verfahren nach einem der Ansprüche 1 bis 4, wobei der Elektronen transferierende Katalysator eine stabile radikalische Aminoxylverbindung ist, vorzugsweise eine stabile radikalische Aminoxylverbindung, die aus der aus 2,2,6,6-Tetramethylpiperidinyloxyl ("TEMPO") und Derivaten davon bestehenden Gruppe ausgewählt ist.

7. Verfahren nach einem der Ansprüche 1 bis 6, wobei das Verfahren nicht von Bestrahlung mit Licht abhängt.

8. Verfahren nach einem der Ansprüche 1 bis 4, wobei der Elektronen transferierende Katalysator ein Übergangsmetall-Koordinationskomplex ist, vorzugsweise ein Übergangsmetall-Koordinationskomplex von Cu, Ru und Ir, mehr bevorzugt ein Übergangsmetall-Koordinationskomplex, der aus der aus Cu(MeCN)₄PF₆, Ru(bpy)₃(PF₆)₂, [Ir(dF(CF₃)ppy)₂(dtbbpy)]PF₆ und Ir(ppy)₃ bestehenden Gruppe ausgewählt ist.

9. Verfahren nach Anspruch 8, wobei der Elektronen transferierende Katalysator ein Photokatalysator ist und das Verfahren unter Bestrahlung mit Licht durchgeführt wird, wobei das Licht sichtbares Licht mit einer Wellenlänge λ im Bereich von 380 nm bis 700 nm ist.

10. Verfahren nach einem der Ansprüche 8 und 9, wobei der Elektronen transferierende Katalysator aus der aus Ru(bpy)₃(PF₆)₂, [Ir(dF(CF₃)ppy)₂(dtbbpy)]PF₆ und Ir(ppy)₃ bestehenden Gruppe ausgewählt ist und vorzugsweise Ru(bpy)₃(PF₆)₂ ist.

11. Verfahren nach einem der Ansprüche 1 bis 10, wobei der Elektronen transferierende Katalysator in unterstöchiometrischen Mengen verwendet wird.

12. Verfahren nach einem der Ansprüche 1 bis 11, wobei das Aren oder Heteroaren in überschüssigen molaren Mengen in Bezug auf das Peroxidreagens gemäß der allgemeinen Formel (I) verwendet wird.

13. Verfahren nach einem der Ansprüche 1 bis 12, wobei das Verfahren in Gegenwart eines organischen Lösungsmittels durchgeführt wird, wobei das Lösungsmittel ein polares aprotisches Lösungsmittel ist, das aus der aus Acetonitril (MeCN), Aceton, *N*,*N*-Dimethylformamid (DMF), Dimethylsulfoxid (DMSO), Tetrahydrofuran (THF), Diethylether, *iso*-Propylether (IPE), Methyl-*tert*-butylether (MTBE), 1,4-Dioxan, Ethylacetat (EtOAc), Dichlormethan (DCM), 1,2-Dichlorethan (1,2-DCE), Chloroform und Mischungen davon bestehenden Gruppe ausgewählt ist; oder in Abwesenheit eines Lösungsmittels.

14. Verfahren nach einem der Ansprüche 1 bis 13, wobei das Verfahren in Gegenwart eines Additivs durchgeführt wird, wobei das Additiv ein Salzadditiv ist, das aus der aus Alkali- und Erdalkalimetallsulfaten, Alkali- und Erdalkalimetallcarbonaten und - hydrogencarbonaten, Alkali- und Erdalkalimetallphosphaten, -hydrogenphosphaten und -dihydrogenphosphaten sowie Alkali- und Erdalkalimetallhalogeniden bestehenden Gruppe ausgewählt ist.

15. Verfahren zur Herstellung pharmazeutischer oder agrochemischer Verbindungen oder Bausteine für die Synthese pharmazeutischer und agrochemischer Verbindungen, umfassend das Verfahren nach einem der Ansprüche 1 bis 14.

## Revendications

1. Procédé de préparation d'arènes et d'hétéroarènes substitués par des groupes (CₙF₂ₙ₊₁)O, **caractérisé en ce que**
un réactif peroxyde selon la formule générale (I) suivante
(OCₙF₂ₙ₊₁)₂ (I),
est fragmenté en présence d'un catalyseur de transfert d'électrons avec formation d'un radical (CₙF₂ₙ₊₁)O, et ledit radical (CₙF₂ₙ₊₁)O substitue alors une liaison C-H d'un arène ou hétéroarène par un groupe (CₙF₂ₙ₊₁)O,
dans lequel, dans les formules ci-dessus, n est un nombre entier compris entre 1 et 4.

2. Procédé de perfluoroalcoxylation directe d'arènes et d'hétéroarènes avec un groupe (CₙF₂ₙ₊₁)O, **caractérisé en ce que**
un réactif peroxyde selon la formule générale (I) suivante
(OCₙF₂ₙ₊₁)₂ (I),
est fragmenté en présence d'un catalyseur de transfert d'électrons avec formation d'un radical (CₙF₂ₙ₊₁)O, et ledit radical (CₙF₂ₙ₊₁)O substitue alors une liaison C-H d'un arène ou d'un hétéroarène par un groupe (CₙF₂ₙ₊₁)O,
dans lequel, dans les formules ci-dessus, n est un nombre entier compris entre 1 et 4.

3. Procédé selon l'une quelconque des revendications 1 et 2, dans lequel n est 1 ou 4, de préférence 1.

4. Procédé selon l'une quelconque des revendications 1 à 3, dans lequel l'hétéroarène est une pyridine, de préférence une pyridine choisie dans le groupe des aminopyridines, des halopyridines et des cyanopyridines.

5. Procédé selon l'une quelconque des revendications 1 à 4, dans lequel le catalyseur de transfert d'électrons est un sel de métal (de transition), de préférence un sel de métal de transition de Cu, Fe, Ti, et plus préférablement un sel de métal de transition choisi dans le groupe constitué de Cu₂O, CuCl, CuCl₂, CuBr, Cul, CuSCN, CuSO₄, CuCO₃, FeSO₄ et TiCl₃.

6. Procédé selon l'une quelconque des revendications 1 à 4, dans lequel le catalyseur de transfert d'électrons est un composé radicalaire aminoxyle stable, de préférence un composé radicalaire aminoxyle stable choisi dans le groupe constitué de 2,2,6,6-tétraméthylpipéridinyloxyle ("TEMPO") et ses dérivés.

7. Procédé selon l'une quelconque des revendications 1 à 6, dans lequel le procédé ne dépend pas d'une irradiation lumineuse.

8. Procédé selon l'une quelconque des revendications 1 à 4, dans lequel le catalyseur de transfert d'électrons est un complexe de coordination d'un métal de transition, de préférence un complexe de coordination d'un métal de transition choisi parmi Cu, Ru et Ir, et plus préférentiellement un complexe de coordination d'un métal de transition choisi dans le groupe constitué par Cu(MeCN)₄PF₆, Ru(bpy)₃(PF₆)₂, [Ir(dF(CF₃)ppy)₂(dtbbpy)]PF₆ et Ir(ppy)₃.

9. Procédé selon la revendication 8, dans lequel le catalyseur de transfert d'électrons est un photocatalyseur et le procédé est effectué sous irradiation lumineuse, la lumière étant une lumière visible ayant une longueur d'onde λ dans la plage comprise entre 380 nm et 700 nm.

10. Procédé selon l'une quelconque des revendications 8 et 9, dans lequel le catalyseur de transfert d'électrons est choisi dans le groupe constitué de Ru(bpy)₃(PF₆)₂, [Ir(dF(CF₃)ppy)₂(dtbbpy)]PF₆ et Ir(ppy)₃, et est de préférence Ru(bpy)₃(PF₆)₂.

11. Procédé selon l'une quelconque des revendications 1 à 10, dans lequel le catalyseur de transfert d'électrons est utilisé en quantités sous-stœchiométriques.

12. Procédé selon l'une quelconque des revendications 1 à 11, dans lequel l'arène ou l'hétéroarène est utilisé en quantités molaires excessives par rapport au réactif peroxyde selon la formule générale (I).

13. Procédé selon l'une quelconque des revendications 1 à 12, dans lequel le procédé est effectué en présence d'un solvant organique, le solvant étant un solvant aprotique polaire choisi dans le groupe constitué de l'acétonitrile (MeCN), de l'acétone, du *N,N-*diméthylformamide (DMF), du diméthylsulfoxyde (DMSO), du tétrahydrofurane (THF), de l'éther diéthylique, l'éther iso-propylique (IPE), l'éther méthyl-*tert*-butylique (MTBE), le 1,4-dioxane, l'acétate d'éthyle (EtOAc), le dichlorométhane (DCM), le 1,2-dichloroéthane (1,2-DCE), le chloroforme et leurs mélanges ; ou en l'absence d'un solvant.

14. Procédé selon l'une quelconque des revendications 1 à 13, dans lequel le procédé est effectué en présence d'un additif, cet additif étant un additif salin choisi dans le groupe constitué des sulfates de métaux alcalins et alcalino-terreux, des carbonates et hydrogénocarbonates de métaux alcalins et alcalino-terreux, des phosphates, hydrogénophosphates et dihydrogénophosphates de métaux alcalins et alcalino-terreux, et des halogénures de métaux alcalins et alcalino-terreux.

15. Procédé de préparation de composés pharmaceutiques ou agrochimiques ou d'éléments constitutifs pour la synthèse de composés pharmaceutiques et agrochimiques, comprenant le procédé selon l'une quelconque des revendications 1 à 14.
